(19) Europäisches Patentamt
European Patent Office
Office européen des brevets

(11) Numéro de publication: **0 012 701**
**B1**

(12) # FASCICULE DE BREVET EUROPEEN

(45) Date de publication du fascicule du brevet: **06.06.84**

(51) Int. Cl.³: **C 08 L 27/06**

(21) Numéro de dépôt: **79420064.2**

(22) Date de dépôt: **04.12.79**

(54) **Compositions de PVC et de polyuréthane à usage médical, procédé pour leur préparation, et objets mis en forme obtenus à partir de ces compositions.**

(30) Priorité: **15.12.78 FR 7836065**

(43) Date de publication de la demande:
**25.06.80 Bulletin 80/13**

(45) Mention de la délivrance du brevet:
**06.06.84 Bulletin 84/23**

(84) Etats contractants désignés:
**BE CH DE GB IT NL SE**

(56) Documents cités:
**DE - A - 2 642 616**
**DE - A - 2 706 390**
**DE - B - 2 503 182**
**FR - A - 2 323 737**
**FR - A - 2 323 738**
**GB - A - 1 350 384**
**US - A - 3 655 814**
**US - A - 3 755 218**
**US - A - 3 853 804**

**Le dossier contient des informations techniques présentées postérieurement au dépôt de la demande et ne figurant pas dans le présent fascicule.**

(73) Titulaire: **RHONE-POULENC RECHERCHES**
**25 Quai Paul Doumer**
**F-92408 Courbevoie Cedex (FR)**

(72) Inventeur: **Pusineri, Christian**
**33, rue des Fleurs Sérézin-du-Rhône**
**F-69360 Saint-Symphorien-d'Ozon (FR)**
Inventeur: **Goletto, Jean**
**"Le Joli Mai" 2, rue Tramier**
**F-69130 Ecully (FR)**

(74) Mandataire: **Vogt, Bernard et al,**
**RHONE-POULENC RECHERCHES Service Brevets Chimie et Polymères Centre de Recherches de Saint-Fons B.P. 62**
**F-69192 St-Fons Cédex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 012 701 B1

## Description

La présente invention concerne des polymères pouvant être mis sous forme d'objets conformés et pouvant avoir un usage dans le domaine médical.

On sait que les polymères sont largement et de plus en plus utilisés dans le domaine médical en vue des applications les plus nombreuses; c'est ainsi que l'on s'en sert pour tous les matériaux devant être en contact avec le sang ou d'autres liquides biologiques; plus précisément on peut citer le besoin de tels produits (appelés "plastiques médicaux") dans la fabrication des flacons de stockage, des poches à sang, des tubes, des sondes, des canules, des cathéters et de tout matériel utile soit dans les opérations de transfusion de sang et de perfusions, soit dans les systèmes de rein et poumon artificiels. Des polymères à propriétés semblables sont recherchés dans d'autres domaines où l'on a aussi besoin de mettre des polymères en contact avec un liquide biologique, par exemple le lait dans le domaine alimentaire.

L'usage de polymères à base du chlorure de vinyle s'est passablement développé en raison de divers avantages liés à la nature de ces produits (en particulier leur coût modéré); malheureusement le polychlorure de vinyle lui-même n'est pas assez souple et si l'on veut le plastifier, les plastifiants connus, notamment les phtalates, ont beaucoup trop tendance à donner lieu aux phénomènes d'exsudation et surtout de lixiviation, ce qui est néfaste par le fait du risque d'accumulation de plastifiant dans le liquide biologique ou le corps humain. La lixiviation des plastifiants du polychlorure de vinyle se produit simplement par extraction de ces plastifiants lorsque le polymère est en contact avec des liquides biologiques.

Un objet de la présente invention est de fournir des polymères utilisables pour être en contact prolongé avec des liquides biologiques, notamment le sang, le plasma, le lait, les solutions de protéines.

Un autre objet de l'invention est de fournir des polymères plastiques du chlorure de vinyle.

Un autre objet de l'invention est de fournir des polymères plastifiés par des plastifiants non lixiviables.

Un autre objet de l'invention est de fournir des polymères héparinés, soit dans la masse, soit après leur mise en forme.

Un autre objet de l'invention est de fournir des polymères héparinés ne larguant que lentement l'héparine qu'ils contiennent.

L'invention concerne donc premièrement des compositions polymériques caractérisées en ce qu'elles comprennent un mélange de polymère du chlorure de vinyle et de polyétheruréthane à groupes amine tertiaire et/ou ammonium.

La proportion respective des constituants dans le mélange peut varier dans de larges limites, par exemple 1 à 99% en poids de polyétheruréthane par rapport à l'ensemble du mélange. Toutefois on préfère que cette proportion de polyuréthane soit comprise entre 20 et 60%.

Comme polymère de chlorure de vinyle on utilise surtout les homo et les copolymères ayant au moins 60% en poids de restes issus du chlorure de vinyle (c'est-à-dire de motifs chloroéthylène $—CHCl—CH_2—$). Comme copolymères plus spécialement utilisables on peut citer les copolymères du chlorure de vinyle et des esters vinyliques, notamment l'acétate de vinyle. Bien entendu on peut aussi utiliser des terpolymères issus du chlorure de vinyle, d'un monomère non ionique et non ionisable (comme les esters vinyliques) et d'un monomère ionique ou ionisable (comme l'acide maléique); la teneur en ce dernier monomère est généralement limitée à 20%, de préférence à 8% en poids par rapport à l'ensemble des monomères du polymère considéré.

Comme polyétheruréthanes à groupes amine tertiaire et/ou ammonium, on peut utiliser soit des polyétheruréthanes dans lesquels les groupes amine et/ou ammonium sont situés dans une ramification de la chaîne polymérique principale, soit des polyétheruréthanes dans lesquels les groupes amine et/ou ammonium sont dans la chaîne polymérique principale.

Les polyétheruréthanes à groupes amine et/ou ammonium utilisés dans l'invention sont, de préférence, des polyuréthanes dérivés de polyéthers à groupes oxyalcoylène (ce groupe oxyalcoylène pouvant être substitué).

Dans ce qui suit, on utilise les lettres n, m, p, q pour désigner respectivement:

— n le nombre de milliéquivalents de motifs oxyéthylène $—O—CH_2—CH_2—$ pour 100 g de polyuréthane,

— m le nombre de milliéquivalents de groupes amine tertiaire, salifiés ou non, pour 100 g de polyuréthane. Les groupes amine tertiaire, lorsqu'ils sont salifiés, constituent des groupes ammonium tertiaire; les polyuréthanes à groupes ammonium utilisés dans l'invention ont donc des groupes ammonium soit tertiaires, soit quaternaires,

— p le nombre de milliéquivalents de groupes ammonium quaternaire pour 100 g de polyuréthane,

— q le nombre de milliéquivalents de groupes ioniques pour 100 g de polymère du chlorure de vinyle.

Les polyétheruréthanes azotés utilisés dans l'invention et les compositions polymériques qui en dérivent sont donc tels que préférentiellement ils vérifient les relations suivantes:

$$m + p \geq 0{,}5$$

$$p + q \geq \frac{n}{100} - 2{,}5$$

Les polyétheruréthanes azotés utilisés dans l'invention comprennent habituellement une pluralité de motifs récurrents de formule:

—A—NH—CO—O—B—O—CO—NH— (I)

et des motifs récurrents de formule:

—A—NH—CO—Z—NH— (II)

dans lesquelles:

— B est le reste divalent d'un macrodiol de type polyéther de formule: HO—B—OH

— A est le reste divalent d'un diisocyanate aliphatique et/ou cycloaliphatique et/ou aromatique de formule: O=C=N—A—N=C=O,

— Z est un lien valentiel ou un radical divalent tel que:

—NH—NH—CO—;

—NH—$CH_2$—CO—NH—NH—CO—;

ou

—$NR_2$—D—$NR_3$—CO—;

ou

—O—M—O—CO—;

$R_2$, et $R_3$ et M sont tels que $NHR_2$—D—$NHR_3$ soit une diamine primaire ou secondaire et HO—M—OH un diol, dans lesquels:

— $R_2$ et $R_3$, identiques ou différents, sont des atomes d'hydrogène ou des radicaux alcoyles inférieurs;

— D est une chaîne hydrocarbonée renfermant de 2 à 12 atomes de carbone, un cycle hydrocarboné à 5 ou 6 atomes de carbone ou un hétérocycle azoté à 5 ou 6 maillons renfermant 1 ou 2 atomes d'azote, les chaînes et cycles hydrocarbonés étant saturés ou insaturés, non substitués ou substitués par un ou deux radicaux alcoyles inférieurs ou par un radical dialcoylamino ou par un hétérocycle azoté à 5 ou 6 chaînons rattaché par un atome d'azote, deux des chaînes ou cycles pouvant être reliés entre eux par un groupement alcoylimino, les hétérocycles pouvant être non substitués ou substitués, en particulier sur un atome d'azote, par un radical alcoyle inférieur;

— M est un radical hydrocarboné aliphatique renfermant 2 à 12 atomes de carbone, linéaire ou ramifié, saturé ou à insaturation éthylénique ou acétylénique, non substitué ou substitué par un ou deux radicaux alcoyles inférieurs ou par un radical dialcoylamino, ininterrompu ou interrompu par un radical alcoylimino.

Les motifs de formule I et II se rattachent les uns aux autres de manière qu'un atome de carbone terminal d'un motif est lié à un atome d'azote terminal d'un autre motif.

Dans l'invention, les groupes amine et/ou ammonium peuvent donc se trouver notamment dans les radicaux B, A, D ou M, mais de préférence B.

Les polyétheruréthanes sont préparés de manière habituelle; et procédé le plus courant consiste à réaliser un macrodiisocyanate à partir d'un macrodiol et d'un diisocyanate; comme macrodiol on utilise par exemple un poly(oxyalcoylène)glycol, notamment un poly(éthylène)glycol ou un poly(propylène)-glycol. Comme diisocyanate on préfère utiliser le diisocyanato-1,6 hexane, les diisocyanato toluènes, les bis(isocyanato cyclohexyl)méthane ou propane, les bis(isocyanato phényl)méthane ou propane.

Ces macrodiisocyanates sont ensuite couplés par des agents de couplage comme l'eau, l'hydrazine, l'hydrazine aminoacétique, les diamines ou les diols.

Le plus souvent le polyuréthane est préparé à partir de réactifs comprenant un group amine tertiaire soit dans le macrodiol, soit dans le diisocyanate, soit dans l'agent de couplage. Une fois le polyuréthane réalisé, on peut quaterniser la fonction amine tertiaire, ce qui fournit donc un polyuréthane à groupes amine tertiaire et/ou ammonium.

Lorsque, selon un mode préféré, on prépare un polyuréthane à groupes amine et/ou ammonium

situées dans la partie polyéther (de préférence polyoxyalcoylène) de la macromolécule de polyuréthane, ce polyuréthane est préparé à partir d'un macrodiol contenant un groupe amine tertiaire. Ainsi selon une variante commode à pratiquer, ce macrodiol est préparé à partir de diols et/ou oxyde d'alcoylène et de diol à fonction amine tertiaire comme par exemple les alcoyl bis(hydroxyalcoyl)-amine, notamment la N-éthyldiéthanolamine.

Les polyétheruréthanes à groupes ammonium sont connus par la demande allemande DOS 2 642 616 et par les brevets US 3 655 814, 3 755 218, 3 853 804.

Les compositions non héparinées selon l'invention sont généralement solubles dans les solvants des polymères du chlorure de vinyle, notamment le diméthylformamide (DMF), le tétrahydrofuranne (THF), et aussi les cétones pour les compositions contenant des copolymères du chlorure de vinyle.

On peut donc réaliser ces compositions et les objets conformés à base de ces compositions selon l'invention par évaporation de solutions de ces compositions; on peut aussi obtenir des objets conformés comprenant un support revêtu de compositions selon l'invention en enduisant un objet conformé en un matériau quelconque à l'aide d'une couche liquide d'enduction puis en évaporant cette couche, cette couche liquide d'enduction étant constituée de solutions de compositions selon l'invention. Un tel procédé est particulièrement adapté pour l'enduction interne de tuyaux (cathéters notamment) mais, bien entendu, on peut considérer des objets conformés de forme tout autre que celle des tuyaux, comme par example les récipients et les surfaces planes.

On peut encore obtenir des objets conformés à partir d'une pâte réalisée à l'aide de compositions selon l'invention contenant un agent gonflant ou une quantité de solvant insuffisante pour en dissoudre la totalité et en extrudant ce mélange composition/solvant ou gonflant.

Le solvant ou gonflant utilisé doit être solvant ou gonflant à la fois pour le polymère du chlorure de vinyle et pour le polyuréthane à groupes amine ou ammonium ou bien gonflant du mélange des deux polymères réunis. comme solvant utilisable on peut citer le diméthylformamide. Dans certains cas, on peut utiliser des solvants plus légers et plus volatiles, comme l'acétone par exemple, ce qui permet d'éliminer plus facilement ce solvant dans le but d'obtenir une composition selon l'invention à l'état sec. Lorsque l'on utilise des polyuréthanes couplés à l'éthylèneglycol (HO—M—OH mentionné plus haut) et des copolymères du chlorure de vinyle et d'acétate de vinyle, on peut alors utiliser de tels solvants légers. Les pâtes à base de compositions selon l'invention sont généralement extrudables à température assez basse (moins de 100°C).

Les matériaux ou objets conformés selon l'invention, constitués de compositions selon l'invention ont une souplesse améliorée (par rapport au matériau correspondant sans polyuréthane) même à température ambiante; ils ont également de bonnes propriétés antistatiques; ils possèdent l'avantage d'éviter l'emploi de plastifiants exsudables ou lixiviables au contact des liquides biologiques ou physiologiques. Les polymères du chlorure de vinyle et les polyétheruréthanes à groupes amine et/ou ammonium étant généralement compatibles surtout lorsqu'ils ont été choisis selon les indications données plus haut, les matériaux ou objets qui sont issus des compositions selon l'invention (notamment les compositions préférées) ont généralement de bonnes propriétés mécaniques et une bonne transparence; en outre ils ont aussi une bonne hémocompatibilité. Leur nature permet de les mettre en oeuvre très commodément. Leur perméabilité à la vapeur d'eau les rend intéressants dans bien des applications réquerant cette qualité.

L'invention a encore pour objet des compositions héparinées à base de polymère du chlorure de vinyle. Les compositions sont caractérisées en ce qu'elles comprennent un polymère du chlorure de vinyle, un polyétheruréthane à groupe ammonium, de préférence quaternaire, et de l'héparine.

Dans ces compositions héparinées, les proportions relatives de polymère de chlorure de vinyle et de polyétheruréthane sont les mêmes que celles indiquées précédemment pour les compositions non héparinées, mais de préférence ces polymères sont choisis de façon que $p_1—q_1 > 0$, $p_1$ étant le produit de p que multiplie le pourcentage pondéral du polyétheruréthane dans la mélange, divisé par 100; $q_1$ étant le produit de q que multiplie le pourcentage pondéral du polymère de chlorure de vinyle à sites ioniques dans le mélange, divisé par 100.

Quant à la proportion pondérale d'héparine, elle est habituellement comprise entre 0,5 et 35% en poids par rapport à l'ensemble des polymères, de préférence entre 1 et 15%.

Dans les compositions selon l'invention et dans ce qui suit, lorsque le terme héparine est utilisé, il est bien entendu que ce terme inclut les formes d'héparine modifiée, ses sels par exemple.

Les compositions héparinées selon l'invention peuvent être obtenues par des procédés similaires à ceux déjà décrits pour les compositions non héparinées. Toutefois, l'héparine étant le plus généralement peu ou pas compatible avec les polymères du chlorure de vinyle seul, il est recommandé d'introduire l'héparine avec le ou en présence de polyétheruréthane à sites ammonium dans le polymère du chorure de vinyle. Ainsi selon un mode avantageux, on mélange une solution de polymère du chlorure de vinyle avec une solution d'héparine et de polyétheruréthane, les deux solvants des deux solutions étant similaires ou tout au moins miscibles. Selon un autre mode avantageux, on ajoute la solution d'héparine à la solution du mélange polyétheruréthane + polymère du chlorure de vinyle. Un procédé particulièrement avantageux de réalisation de compositions héparinées selon l'invention et notamment de compositions conformées, consiste à réaliser une pâte et à conformer cette pâte par exemple par extrusion. La pâte peut s'obtenir par mélange des constituants avec une quantité de solvants insuffisante pour obtenir une solution. On peut aussi obtenir une pâte en éliminant partiellement le solvant d'une solution. Pour solubiliser l'héparine ou les mélanges héparine + polyétheruréthane à groupes amine et/ou ammonium, on utilise généralement des mélanges d'eau et de diméthylsulfoxyde, ou d'eau et de diéthylèneglycol ou d'eau et de diméthylformamide. Pour réaliser des pâtes en compositions héparinées selon l'invention, on se sert avantageusement de solvant ou gonflant de ces compositions, par exemple le diméthylformamide, les mélanges eau + diméthylformamide ou eau + diéthylèneglycol ou eau + diméthylsulfoxyde.

Avec les compositions héparinées selon l'invention il est assez avantageux de les mettre en oeuvre avec un solvant volatil (par exemple l'acétone) pour pouvoir évaporer ce solvant sans nuire à l'héparine (notamment par dégradation). Comme indiqué précédemment, les compositions selon l'invention dérivées de copolymères du chlorure de vinyle et de polyétheruréthanes couplés à l'éthylèneglycol se prêtent particulièrement bien à l'utilisation de solvant volatil pour la réalisation de pâtes extrudables.

Pour obtenir une telle pâte, il est généralement, avantageux de commencer par faire une solution des trois constituants du mélange, de manière à obtenir un mélange bien homogène, puis, après avoir éliminé le solvant par exemple par précipitation avec un non solvant, d'ajouter le solvant volatil donnant naissance à la pâte. Comme solvant volatil on préfère l'acétone. Comme solvant du mélange ternaire hépariné, on préfère utiliser les mélanges eau/solvant polaire aprotique notamment eau/DMF, eau/DMSO, eau/DEG, eau/DMF (DMF = diméthylformamide, DMSO = diméthylsulfoxide, DEG = diéthrylèneglycol).

Ce procédé peut être modifié en réalisant la première solution des constituants du mélange, avec seulement le polyuréthane et l'héparine; dans ce cas l'adjonction du polymère du chlorure de vinyle peut n'intervenir qu'ultérieurement par malaxage à l'état pâteux et en présence de solvant volatil, de l'ensemble des trois constituants du mélange.

Les compositions héparinées selon l'invention présentent une hémocompatibilité particulièrement bonne, leur antithrombogénicité est également très bonne, notamment en raison de la lixiviation très lente de l'héparine. Leur utilisation pour réaliser des objets en contact avec le sang (tubes, poches à sang, vaisseaux artificiels, cathéters) est donc particulièrement recommandée. Les compositions héparinées pouvant être mises en oeuvre sous forme de pâte, et surtout de pâte utilisant un solvant volatil, sont préférées.

Bien que ci-avant il ne soit question que de compositions héparinées dans la masse, l'invention concerne également des objets conformés, à base de polymères de chlorure de vinyle et de polyétheruréthane comprenant des sites ammonium de préférence quaternaire, héparinés en surface. Dans ce cas les quantités d'héparine utilisées sont fortement réduites.

Les exemples suivants, 1 à 25, illustrent l'invention et montrent comment elle peut être mise en oeuvre. Plus précisément, les exemples 1 à 4 illustrent simplement la préparation de polyétheruréthanes à groupes amine tertiaire et/ou ammonium quaternaire; les exemples 5 à 13 illustrent des mélanges selon l'invention de polymères du chlorure de vinyle et de polyétheruréthanes à groupes amine tertiaire salifiés ou non salifiés et/ou à groupes ammonium quaternaire; les exemples 14 à 16 illustrent des mélanges héparinés selon l'invention de polymère du chlorure de vinyle et de polyétheruréthane à groupes amine tertiaire et/ou ammonium.

Exemple 1

*Preparation de polyetherurethane non quaternise:* dans un autoclave de 7 500 $cm_3$, muni d'une atmosphère d'azote et d'un système d'agitation, on charge 333 g de N-éthyldiéthanolamine et 8,32 g d'hydroxyde de potassium; on chauffe à 95°C jusqu'à dissolution complète. Après refroidissement on déshydrate la masse réactionnelle à 80—82°C sous une pression absolue de 1 733 Pa (13 mm Hg), puis on élève la température à environ 111°C et injecte l'oxyde de propylène, la pression étant maintenue entre $4 \times 10^5$ Pa et $5 \times 10^5$ Pa (4 et 5 bars). La quantité d'oxyde de propylène introduite au bout de 8 heures est de 2 480 g; on maintient encore la masse réactionnelle à cette température pendant 2 heures, puis élimine l'oxyde de propylène n'ayant pas réagi (120 g) par distillation sous vide. Le polyéther ainsi obtenu contient 0,936 milliéquivalent/gramme d'azote tertiaire. On neutralise ce polyéther par addition d'acide chlorhydrique jusqu'à pH 6—7, puis déshydrate la masse réactionnelle par chauffage à 80°C sous pression absolue de 133,3 à 266,6 Pa (1 à 2 mm Hg) et sépare, par filtration, le chlorure de potassium formé.

A 930,7 g de ce polyéther salifié, porté à 80°C, on ajoute en une seule fois sous atmosphère de gaz inerte, 422,5 g de diisocyanato-4,4', diphénylméthane et maintient le mélange à cette température sous agitation pendant 45 minutes. Le macrodiisocyanate obtenu est refroidi et dissous dans 750 $cm^3$ de diméthylformamide.

On prépare ensuite une solution de 37,6 g d'hydrazide amino-acétique dans 1 740 g de diméthylformamide par chauffage à 60°C. Dans cette solution refroidie, on introduit en 1 heure 45 minutes 1 015 g de la solution de macrodiisocyanate précédemment préparée, puis on dilue par addition de 1 890 g de diméthylformamide. La solution de polyuréthane ainsi obtenue est versée lentement et sous forte agitation dans un mélange de 8 kg de glace et 24 kg d'eau contenant 128 g d'hydroxyde de sodium, afin de précipiter le polymère. Celui-ci est essoré, lavé à l'eau jusqu'à neutralité,

séché sous vide à 40°C, puis sur anhydride phosphorique à température ambiante jusqu'à poids constant. On obtient ainsi 682 g de polyuréthane contenant 0,583 milliéquivalent/g (mg/g) d'azote tertiaire (dosage acidimétrique).

*Preparation du polyetherurethane quaternise:* on dissout 256 g du polyétheruréthane précédemment décrit dans 1 344 g de diméthylformamide et l'on ajoute à cette solution 85 g d'iodure de méthyle. Le mélange est agité 15 minutes à température ambiante puis 9 heures 30 minutes à 46°C. Après refroidissement on le coule dans un mélange de 7,5 kg de glace et 25 kg d'eau sous forte agitation; on sépare le polymère précité par filtration, le lave à l'eau puis au méthanol et sèche comme précèdemment à poids constant. Le taux de quaternisation, déterminé par dosage acidimétrique est de 100%. Le polymère contient 0,538 milliéquivalent d'ammonium quaternaire par gramme (meq/g).

Exemple 2

*Preparation de polyetherurethane non quaternise:* dans un réacteur de 1 litre, muni d'une atmosphère d'azote, d'un système d'agitation et d'un réfrigérant à reflux, on charge 300 g d'un polyoxyéthylèneglycol (commercialisé sous la marque CARBOWAX 1 500) de masse moléculaire 1 500. Après fusion, la masse réactionnelle est déshydratée à 100°C sous pression absolue de 133,3 Pa (1 mm Hg). On ramène à pression atmosphérique avec de l'azote et la température est abaissée à 80°C. On charge 0,4 g de malonitrile puis, 15 minutes après, on introduit 100 g de diisocyanato-4,4'diphénylméthane. La température de la masse réactionnelle est maintenue à 80°C pendant 3 heures. Le macrodiisocyanate obtenu est refroidi et dissous dans 250 $cm^3$ de diméthylformamide.

On prépare ensuite une solution de 23,7 g d'hydrazide aminoacétique dans 1 250 g de diméthylformamide. Dans cette solution refroidie, on coule en 4 heures un mélange de 258 g de solution à 65% dans le diméthylformamide du macrodiisocyanate préparé selon l'exemple 1 et de 578 g de solution à 63% dans le diméthylformamide du macrodiisocyanate précédemment décrit au début de cet exemple 2. La solution de copolyuréthane ainsi obtenue est versée lentement et sous forte agitation dans 30 litres d'eau contenant 600 g de chlorure de sodium et 120 g d'hydroxyde de sodium à + 5°C, afin de précipiter le polymère. Celui-ci est essoré, lavé à l'eau jusqu'à neutralité et disparition des ions chlorures, séché sous vide à 40°C, puis sur anhydride phosphorique à température ambiante jusqu'à poids constant. On obtient ainsi 460 g de copolyuréthane contenant 0,189 milliéquivalent/g d'azote tertiaire et 1 124 meq de motifs oxyéthylène pour 100 g de polyuréthane.

*Preparation du copolyurethane quaternise:* dans un réacteur de 5 litres, équipé d'un système d'agitation, on introduit 330 g de copolyuréthane qui vient d'être ainsi préparé et 3 300 $cm^3$ d'acétone. Sous atmosphère d'azote, on charge 19,7 g d'iodure de méthyle, bouche le réacteur et agite pendant 20 heures à température ambiante. Le polymère est séparé par filtration, lavé à l'acétone puis à l'éther et séché à poids constant comme précédemment. Le taux de quaternisation est de 100%. Le polymère contient 0,184 milliéquivalent/g d'ammonium quaternaire et 1 094 meq de motifs oxyéthylène pour 100 g de polyuréthane.

Exemple 3

*Preparation de polyurethane non quaternise:* dans un réacteur de 5 litres équipé d'un système d'agitation, d'une atmosphère d'azote et d'un réfrigérant à reflux, on charge 16,7 g d'éthylèneglycol, 0,4 g de dilaurate de dibutylétain, 950 g de diméthylformamide. On agite et coule en une heure 792 g d'une solution à 55 % dans le diméthylformamide de macrodiisocyanate préparé selon l'exemple 1. La température de la masse réactionnelle est portée à 70—75°C pendant une heure et on termine lentement l'addition de la solution de macrodiisocyanate. La solution de polyuréthane ainsi obtenue est versée sous forte agitation dans 20 litres d'eau contenant 400 g de chlorure de sodium et 80 g d'hydroxyde de sodium à + 5°C, afin de précipiter le polymère. Celui-ci est essoré, lavé à l'eau jusqu'à neutralité et disparition des ions chlorures, séché sous vide à 40°C puis sur anhydride phosphorique à température ambiante jusqu'à poids constant. On obtient ainsi 450 g de polyuréthane contenant 0,599 milliéquivalent/g d'azote tertiaire.

*Preparation de polyetherurethane quaternise:* le polyétheruréthane non quaternisé préparé ci-avant est quaternisé selon la technique décrite à l'exemple 1. Le polymère obtenu contient 0,561 meq/g d'ammonium quaternaire. Bien entendu les sels d'étain sont éliminés par précipitation du polymère dans un non solvant, mais solvant des sels d'étain.

Exemple 4

*Preparation de polyetherurethane non quaternise:* dans un réacteur de 5 litres, muni d'un système d'agitation, d'une atmosphère d'azote et d'un réfrigérant à reflux, on charge 32,6 g d'éthylèneglycol, 0,97 g de dilaurate de dibutylétain, 2 267 g de diméthylformamide.

Dans cette solution, on coule en 1 heure, 90% d'un mélange contenant 676,8 g d'une solution à 47% dans le diméthylformamide d'un macrodiisocyanate préparé selon l'exemple 1 et 1 144 g d'une solution à 54% dans le diméthylformamide d'un macrodiisocyanate préparé selon la technique décrite au premier paragraphe de l'exemple 2.

La température de la masse réactionnelle est portée à 70—75°C pendant 1 heure, on termine lentement l'addition de la solution des deux macrodiisocyanates, puis on ajoute au milieu réactionnel,

une solution de 26 g de diisocyanato-4,4'diphénylméthane dans 80 g de diméthylformamide. La solution de copolyuréthane ainsi obtenue est versée, sous forte agitation, dans 50 litres d'eau contenant 1 000 g de chlorure de sodium et 200 g d'hydroxyde de sodium à + 5°C, afin de précipiter le polymère. Celui-ci est essoré, lavé à l'eau jusqu'à neutralité et disparition des ions chlorures puis lavé au méthanol et séché sous vide à 40°C puis sur anhydride phosphorique à température ambiante jusqu'à poids constant. On obtient ainsi 1 000 g de copolyuréthane contenant 0,195 milliéquivalent/g d'azote tertiaire et 1,066 meq/g de motifs oxyéthylène —O—$OH_2$—$CH_2$.

*Preparation du copolyurethane quaternise:* dans un réacteur de 5 litres, équipé d'un système d'agitation, on introduit 500 g de copolyuréthane préparé au début du présent exemple et 4 litres de méthanol. Sous atmosphère d'argon on charge 27,7 g d'iodure de méthyle, bouche le réacteur et agite pendant 20 heures à température ambiante. Le polymère est séparé par filtration, lavé au méthanol et séché à poids constant comme précédemment.

Le taux de quaternisation est de 77%. Le polymère contient 0,150 milliéquivalent/g d'ammonium quaternaire et 0,042 milliéquivalent/g d'azote tertiaire non salifié.

Exemple 5

Dans un réacteur de 2 litres équipé d'un système d'agitation, on charge 900 g de dimèthylformamide et refroidit à environ 3°C. On introduit alors lentement 100 g d'un polychlorure de vinyle dont l'indice de viscosité (mesure effectuée selon Encyclopedia of Polymer Science and Technology vol. 14, p. 374 ed. 1971) est égal à 80 dl/g. On laisse remonter la température de la masse jusqu'à la température ambiante et on poursuit l'agitation jusqu'à dissolution complète.

Dans un flacon de 125 $cm^3$, on charge 80 g de la solution précédemment préparée et 5,33 g de polyuréthane à groupes amine tertiaire (ni salifiés ni quaternisés) obtenu selon l'exemple 1. Le rapport pondéral entre le chlorure de polyvinyle et le polyuréthane est de 60/40 et la concentration des polymères dans le solvant de 15,6%. Le flacon est secoué pendant 24 heures jusqu'à dissolution complète. La solution obtenue est filtrée et dégazée puis coulée sur une plaque de verre de manière à former un film liquide d'environ 0,4 mm d'épaisseur. Ce film est séché à 50°C sous une pression absolue de 26 660 Pa (200 mm Hg) puis détaché de son support par trempage dans l'eau. Le matériau obtenu est transparent et présente une bonne résistance au déchirement. Ses propriétés mécaniques sont les suivantes:

— résistance à la traction à la rupture: 356 kg/$cm^2$ (363 Bar)
— allongement à la traction à la rupture: 84%

Exemple 6

La préparation du mélange polymère du chlorure de vinyle-polyuréthane est réalisée dans des conditions identiques à celles de l'exemple 5 sauf que le polyuréthane utilisé est le polyuréthane contenant 0,538 milliéquivalent/g de groupes ammonium quaternaire obtenu selon la technique décrite dans l'exemple 1 (préparation du polyuréthane quaternisé).

Le matériau obtenu est transparent et présente une bonne résistance au déchirement et ses propriétés mécaniques sont les suivantes:

— résistance à la traction à la rupture: 427 kg/$cm^2$ (436 Bar)
— allongement à la traction à la rupture: 74%

Exemple 7

La préparation du mélange polymère du chlorure de vinyle-polyuréthane est réalisée dans des conditions identiques à celles de l'exemple 5 sauf que le polyuréthane utilisé est le polyuréthane contenant 0,599 milliéquivalent/g d'azote tertiaire (groupes amine tertiaire non quaternisés) obtenu selon la technique décrite dans l'exemple 3.

Le matériau obtenu est transparent et présente une bonne résistance au déchirement.

Exemple 8

La préparation du mélange polymère du chlorure de vinyle-polyuréthane est réalisée dans des conditions identiques à celles de l'exemple 5 sauf que le polyuréthane utilisé est le polyuréthane à groupes ammonium quaternaire obtenu selon la technique décrite dans l'exemple 3.

La matériau obtenu est transparent et présente une bonne résistance au déchirement.

Exemple 9

La préparation du mélange polymère du chlorure de vinyle-polyuréthane est réalisée dans des conditions identiques à celles de l'exemple 5 sauf que le polyuréthane utilisé est le polyuréthane contenant 0,184 milliéquivalent/g d'azote quaternaire (groupes ammonium quaternaire) obtenu selon la technique décrite dans l'exemple 2 (Préparation de polyuréthane quaternisé).

Le matériau obtenu est transparent et présente une bonne résistance au déchirement et ses propriétés mécaniques sont les suivantes:

— résistance à la traction à la rupture: 374 kg/$cm^2$ (382 Bar)
— allongement à la traction à la rupture: 100%

Exemple 10

La préparation du mélange polymère du chlorure de vinyle-polyuréthane est réalisée dans des conditions identiques à celles de l'exemple 5 sauf que le polyuréthane utilisé est le polyuréthane contenant 0,150 milliéquivalent/g d'azote quaternaire (groupes ammonium quaternaire) et 0,032 milliéquivalent/g d'azote tertiaire obtenu selon la technique décrite dans l'exemple 4 (préparation de polyuréthane quaternisé).

Le matériau obtenu est transparent et présente une bonne résistance au déchirement.

Exemple 11

La préparation du mélange polymère du chlorure de vinyle-polyuréthane est réalisée dans des conditions identiques à celles de l'exemple 5 sauf que l'on introduit, dans la solution du mélange de polymères, 3,1 $cm^3$ d'une solution N d'HCl dans le diméthylformamide, ce qui a pour effet de salifier les sites amine tertiaire (et de les transformer en groupes ammonium tertiaire). Le film obtenu selon la technique décrite à l'exemple 5 est souple et transparent et présente une bonne résistance au déchirement.

Exemple 12

Dans les exemples 5 à 11 on remplace le polychorure de vinyle par un copolymère chlorure de vinyle/acétate de vinyle dont les motifs correspondant à ces deux monomères sont en proportions pondérales respectives 85/15, et dont l'indice de viscosité mesuré selon la norme AFNOR NF T-51013 est de 54 $cm^3/g$ et son coefficient K (tel que défini dans Encyclopedia of Polymer Science and Technology vol. 14, p. 374—5 Ed. 1971) est égal à environ 46.

On obtient des résultats semblables à ceux des exemples 5 à 11, c'est-à-dire que le matériau obtenu (à base d'un mélange de polymère du chlorure de vinyle et de polyuréthane à groupes amine et/ou ammonium quaternaire), sous forme d'un film souple et transparent, est pourvu de bonnes propriétés mécaniques, notamment une bonne résistance au déchirement.

Exemple 13

On utilise comme polymère du chlorure de vinyle un terpolymère chlorure de vinyle/acétate de vinyle/acide maléique. Les motifs récurrents correspondant à ces trois monomères sont en proportions pondérales respectives 84,5/14,4/1,1 (0,189 meq/g de groupes acides). Ce polymère a une masse moléculaire telle que son indice de viscosité (selon la norme AFNOR NF-51013) est d'environ 55 $cm_3/g$ et que son coefficient K (tel que defini dans Encyclopedia of Polymer Science and Technology vol. 14, p. 374—5, Ed. 1971) est égal à environ 46.

On reproduit alors la technique décrite à l'exemple 5 en remplaçant le polymère du chlorure de vinyle décrit par ce terpolymère et en utilisant comme polyuréthane successivement chacun des polyuréthanes non quaternisés et chaucun des polyuréthanes salifiés ou quaternisés décrits dans chacun des exemples 1 à 4.

On obtient des films souples, transparents et pourvus de bonnes propriétés mécaniques, notamment une bonne résistance au déchirement.

Exemple 14

Dans un réacteur équipé d'un système d'agitation on charge 405 g de diméthylformamide, agite et indroduit lentement 90 g de polyuréthane contenant 0,150 milliéquivalent/g d'ammonium quaternaire et 0,042 milliéquivalent/g d'azote tertiaire obtenu selon la technique décrite dans l'exemple 4 (préparation de polyuréthane quaternisé) puis 45 g de terpolymère utilisé à l'exemple 13. L'agitation est poursuivie jusqu'à dissolution complète, la concentration des polymères dans le solvant est alors de 25%.

On prépare parallèlement une solution de 15 g d'héparine (sous forme de sel de sodium) dans 33 $cm^3$ d'eau, qui est versée lentement dans 48 $cm^3$ de diéthylèneglycol. La solution homogène obtenue est versée goutte-à-goutte en une heure dans le mélange de polyuréthane et de copolymère de chlorure de vinyle.

La composition obtenue est coulée lentement dans six litres d'éther éthylique sous forte agitation afin de précipiter le polymère hépariné. Celui-ci est filtré, lavé à l'éther et séché en étuve à vide à 50°C sous une pression absolue de 26 660 Pa (200 mm Hg), jusqu'à poids constant (143,7 g).

Dans un malaxeur on introduit 60 g de la composition héparinée précédemment préparée et ajoute 35 $cm^3$ d'acétone par peties fractions. Le malazage est poursuivi jusqu'à obtention dune pâte homogène. La pâte acétonique à 27% d'acétone ainsi obtenue est extrudée à travers une filière à secteur donnant naissance à un tube fin. L'extrusion s'effectue à 70—80°C sous une pression de $470 \times 10^5$ Pa à $580 \times 10^5$ Pa (470 à 580 bars) avec une vitesse de sortie du tube de la filière d'environ 35 cm/minute. Le tube est ensuite coupé et les morceaux de tube extrudé sont séchés trois jours en position verticale et en atmosphère ambiante, puis sous une pression absolute de 26 660 Pa (200 mm Hg) à 50°C. Les cathéters ainsi préparés ont un diamètre extérieur de 1,9 mm et une épaisseur de paroi de 0,2 mm; mis en contact avec du sang de chien fraîchement prélevé, ils ne provoquent pas la coagulation pendant la durée de l'essai qui a été d'une heure.

Exemple 15

On mélange 50 g de polyuréthane à groupe ammonium quaternaire (préparé selon l'exemple 2) et 50 g de polychlorure de vinyle. Le mélange est dissous dans 400 g de diméthylformamide sous agitation, à 23°C.

On prépare par ailleurs une solution à l'aide de 20 g d'héparine (sous forme de sel de sodium) et de 35 g d'eau, puis 175 g de diéthylèneglycol.

La solution d'héparine est alors ajoutée à la solution de mélange polymérique. On obtient ainsi une solution de composition polymérique héparinée selon l'invention.

Cette solution est utilisée d'une part pour faire des films et d'autre part pour faire un cathéter.

*Preparation de film:* la solution de composition polymérique héparinée est coulée sur une plaque de verre de manière à constituer un film liquide de dimensions 25 cm × 30 cm × 0,1 cm. On sèche 12 heures à 50°C sous pression absolue de 26 660 Pa (200 mm Hg). On lave à l'eau et sèche à nouveau de la même manière.

*Preparation du catheter:* une baguette de verre de longueur 25 cm et de diamètre 3 mm est trempée dans la solution de composition polymérique héparinée. On laisse égoutter à température ambiante une heure, puis on sèche à 50°C pendant une heure sous pression absolue de 26 660 Pa (200 mm Hg). On répète quatre fois ces opérations d'enduction puis on sèche finalement 12 heures à 50°C sous pression réduite à 26 660 Pa (200 mm Hg) [pression absolue].

Le film aussi bien que le cathéter, lorsqu'ils sont mis en contact avec du sang de chien fraîchement prélevé, ne provoquent pas la coagulation du sang pendant la durée de l'essai (une heure).

Exemple 16

On reproduit l'exemple 15 en remplaçant le polychlorure de vinyle d'une part part un copolymère de chlorure de vinyle/acétate de vinyle tel qu'utilisé à l'exemple 12 et d'autre part par un terpolymère tel qu'utilisé à l'exemple 13.

On obtient de la même manière des films et des cathéters ne donnant pas lieu à coagulation du sang.

Exemple 17

17-1. — *Preparation d'un macrodiisocyanate popg a partir de polyoxypropyleneglycol a sites amines tertiaires salifiees par HCl:*

Dans un réacteur de deux litres équipé d'un système d'agitation, d'un réfrigérant à reflux et d'une surpression d'azote, on introduit 385,7 g de polyéther salifié préparé selon l'exemple 1. Le polyéther salifié est porté à 100°C sous une pression absolue de 133,3 Pa (1 mm Hg) pendant une heure afin de le déshydrater. La température de la masse est ramenée à 80°C et la pression à la pression normale. On ajoute ensuite, sous forme fondue et en une seule fois, 188,2g de bis(4-isocyanato-cyclohexyl)-méthane; la masse est maintenue à 80°C sous agitation pendant 50 heures. Le macrodiisocyanate obtenu est refroidi et dissous dans 325 ml de diméthylformamide.

17-2. — *Preparation d'un macrodiisocyanate a partir d'un polyoxyethyleneglycol:*

Dans un appareillage identique au précédent et parallèlement, on charge 601 g d'un polyoxyéthylèneglycol (commercialisé sous la marque CARBOWAX 1 500) de masse moléculaire 1 500. Après fusion, la masse réactionnelle est déshydratée à 100°C sous pression absolue de 133,3 Pa (1 mm Hg). On ramène à pression atmosphérique avec de l'azote et la température est abaissée à 80°C. On charge ensuite 220,1 g de bis(4-isocyanato-cyclohexyl)méthane sous forme fondue en une seule fois. La température de la masse réactionnelle est maintenue à 80°C pendant 8 heures sous agitation. Le macrodiisocyanate obtenu est refroidi et dissous dans 465 ml de diméthylformamide.

17-3. — *Preparation d'un polyurethane a sites salifies:*

Dans un réacteur de 5 litres muni d'un système d'agitation, d'une surpression d'azote et d'un réfrigérant à reflux, on charge 3 063 g de diméthylformamide et 32,97 g de propane diamine 1—2 préalablement distillé sur potasse.

Dans cette solution à température ambiante, on coule, en 15 minutes, 80% d'un mélange contenant 863,1 g d'une solution à 65% dans le diméthylformamide du macrodiisocyanate préparé selon le paragraphe 17-1. et 440,64 g d'une solution à 65% dans le diméthylformamide du macrodiisocyanate préparé précédemment et décrit dans le paragraphe 17-2. On termine lentement l'addition du reste du mélange en une heure et 30 minutes. On laisse la masse réactionnelle sous agitation et sous une pression d'azote pendant 15 heures.

1/3 de la solution de copolyuréthane ainsi obtenue est versée lentement et sous agitation dans 30 litres d'eau contenant 600 g de chlorure de sodium à + 5°C afin de précipiter le polymère. Celui-ci est essoré, lavé à l'eau jusqu'à disparition des ions chlorures, puis séché sous vide à 30°C.

On obtient ainsi 211 g de copolyuréthane contenant 0,204 milliéquivalent/g d'azote salifé et 10,61 milliéquivalents/g de motifs oxyéthylène.

17-4. — *Preparation d'un polyurethane non quaternise:*

Les 2/3 de la solution de copolyuréthane préparée précédemment sont versés lentement dans 60 litres d'eau contenant 1 200 g de chlorure de sodium et 240 g d'hydroxyde de sodium à + 5°C, afin de précipiter le polyéther et libérer l'amine. Celui-ci est essoré et lavé à l'eau jusqu'à neutralité et disparition des ions chlorures. Le produit est séché par lyophilisation.

On obtient ainsi 590 g de copolyuréthane contenant 0,206 milliéquivalent/g d'azote tertiaire.

17-5. — *Preparation de polyurethane quaternise:*

188 g de polyuréthane non quaternisé préparé ci-avant sont quaternisés selon la technique décrite à l'exemple 4; seul le méthanol étant replacé par l'acétone.

On obtient 183 g de polymère sec à 0,20 milliéquivalent/g d'ammonium quaternaire.

Exemple 18

On mélange 12,5 g de polyuréthane à groupes ammonium quaternaire, préparé selon l'exemple 17, et 12,5 g de polychlorure de vinyle, décrit dans l'exemple 5. Le mélange est dissous dans 290 ml de diméthylformamide, sous agitation pendant 3 heures à 23°C, puis une heure à 80°C.

La solution est filtrée et dégazée puis coulée sur plaque de verre de manière à former un film liquide d'environ 1 mm d'épaisseur. Ce film est séché à 50°C sous une pression absolue de 26 660 Pa (200 mm Hg), puis détaché de son support par trempage dans l'eau. Le film obtenu est transparent et présente une bonne résistance au déchirement. Ses propriétés mécaniques sont les suivantes:

— résistance à la traction à la rupture: 370 kg/cm$^2$ (378 Bar)
— allongement à la traction à la rupture: 270%.

Exemple 19

La préparation de film de polychlorure de vinyle et de polyuréthane à groupes amine tertiaire, obtenu selon l'exemple 17, est réalisée dans des conditions identiques à celles de l'exemple 18. On obtient un matériau transparent, présentant une bonne résistance au déchirement:

— résistance à la traction à la rupture: 310 kg/cm$^2$ (316 Bar)
— allongement à la traction à la rupture: 230%.

Exemple 20

La preparation de film de polychlorure de vinyle et de polyuréthane à groupes amine tertiaire salifiés par HCl, obtenu selon l'exemple 17, est réalisée dans des conditions identiques à celles de l'exemple 18. On obtient un film transparent, présentant une bonne résistance au déchirement:

— résistance à la traction à la rupture: 300 kg/cm$^2$ (306 Bar)
— allongement à la traction à la rupture: 195%.

Exemple 21

Dans les exemples 18 à 20, on remplace le polychorure de vinyle par un terpolymère chlorure de vinyle/acétate de vinyle/acide maléique décrit dans l'exemple 13.

On obtient des résultats semblables à ceux des exemples 18 à 20, c'est-à-dire que les matériaux obtenus (à base d'un mélange du terpolymère de chlorure de vinyle et de polyuréthane à groupes amine et/ou ammonium quaternaire, préparés selon l'exemple 17) sous forme de films souples et transparents, sont pourvus de bonnes propriétés mécaniques.

Exemple 22

On mélange 12,5 g de polyuréthane à groupes ammonium quaternaire préparé selon l'exemple 17-5. et 12,5 g de terpolymère chlorure de vinyle/acétate de vinyle/acide maléique, décrit dans l'exemple 13. Le mélange est dissous sous agitation à 100°C pendant deux heures dans 100 g de diméthylformamide. On prépare par ailleurs une solution à l'aide de 5 g d'héparine (sous forme de sel de sodium) et de 8,75 g d'eau et 43,8 g de diéthylèneglycol.

La solution d'héparine est alors ajoutée à la solution du mélange polymérique préalablement refroidie et ramenée à la température ambiante. On obtient ainsi une solution de composition polymérique héparinée selon l'invention.

Cette solution est utilisée pour faire un film.

*Preparation du film:*

La solution de composition polymérique héparinée est coulée sur une plaque de verre de manière à constituer un film liquide de dimensions 20 cm × 30 cm × 0,1 cm. On sèche 24 heures à 50°C sous pression absolue de 26 660 Pa (200 mm Hg). On lave à l'éther et sèche à nouveau de la même manière. On obtient ainsi un film contenant 16,6% d'héparine.

*Test de coagulation sur le film obtenu:*

On découpe des membranes de diamètre 12 cm, à partir du film obtenu hépariné.

Ces films sont placés dans des cônes en verre, de manière à ce qu'ils épousent la forme de ceux-ci, puis sont remplis de sérum physiologique, le tout étant placé dans un bain régulé à 37°C.

Après avoir réalisé l'equilibre de température, on chasse le sérum physiologique et l'on introduit dans chaque cône 2 ml de sang veineux humain, fraîchement prélevé. Sur le verre, le sang coagule après cinq minutes et 30 secondes de contact. Sur les matériaux héparinés aucune coagulation ne se produit après 1 heure 30 minutes de contact.

Exemple 23

On reproduit l'exemple 22 en remplaçant le terpolymère par le polychorure de vinyle décrit dans l'exemple 5.

On obtient de la même manière des films ne donnant pas lieu à la coagulation du sang.

Exemple 24

Le film préparé à l'exemple 18, dont la capacité de gonflement est de 15% dans l'eau et de 40% dans un mélange éthanol/eau (50—50 en volume), est plongé dans 250 ml d'une solution eau/éthanol (50—50 en volume) à 3% en poids d'héparine (sous forme de son sel de sodium) pendant 16 heures à 50°C. Le film est alors lavé à l'eau et séché à l'eau et séché à 50°C pendant 10 heures, sous une pression absolue de 26 660 Pa (200 mm Hg).

Le film obtenu contient environ 2% en poids d'héparine.

Exemple 25

Dans un réacteur équipé d'un système d'agitation, on charge 90 g de diméthylformamide, on agite et on introduit lentement 10 g de polyuréthane contenant 0,189 milliéquivalent/g d'azote tertiaire préparé selon l'exemple 2.

Après dissolution on coule 2,55 ml d'acide chlorhydrique de normalité 1N afin de salifier le polyuréthane, puis on ajoute lentement 5 g de terpolymère utilisé à l'exemple 13. L'agitation est poursuivie jusqu'à dissolution complète.

On prépare parallèlement une solution d'héparine (sous forme de sel de sodium) contenant 1,67 g d'héparine, 2,9 ml d'eau et 14,55 g de diéthylèneglycol. Cette solution est alors ajoutée en 30 minutes dans le mélange de polyuréthane salifié et de terpolymère de chlorure de vinyle.

La solution héparinée est alors filtrée, dégazée puis coulée sur une plaque de manière à constituer un film liquide de dimensions 25 cm × 30 cm × 0,1 cm. On sèche 24 heures à 50°C sous pression absolue de 26 660 Pa (200 mm Hg). On obtient un film homogène souple, transparent.

Aucune coagulation ne se produit après 1 heure 30 minutes de contact de sang avec ce film, le test ayant lieu dans les conditions définies à l'exemple 22.

**Revendications**

1. Compositions polymères homogènes utilisables notamment pour la réalisation d'objets conformés souples, généralement transparents et ayant une bonne compatibilité avec les liquides biologiques et physiologiques, lesdites compositions étant caractérisées en ce qu'elles sont telles que celles obtenues en mélangeant un polyétheruréthane à groupe amine tertiaire et/ou ammonium, ledit polyétheruréthane ne comportant pas d'enchaînement polyester, et un polymère de chlorure de vinyle,

— lorsque ce mélange est obtenu en dissolvant complètement les deux polymères, en milieu solvant, puis en évaporant le solvant,

— ou lorsque le mélange est obtenu à partir d'une pâte avec un agent gonflant ou une quantité de solvant insuffisante pour dissoudre la totalité des polymères, puis en extrudant cette pâte.

2. Compositions selon la revendication 1, caractérisée en ce que la proportion de polyuréthane est comprise entre 20 et 60% en poids.

3. Compositions selon l'une des revendications 1 à 2, caractérisées en ce que le polymère du chlorure de vinyle comprend au moins 60% en poids de motifs chloroéthylène.

4. Compositions selon l'une des revendications 1 à 3, caractérisées en ce que le polymère du chlorure de vinyle est un homopolymère ou chlorure de vinyle ou un copolymère chlorure de vinyle-ester vinylique.

5. Compositions selon l'une des revendications 1 à 4, caractérisées en ce que les polyétheruréthanes sont des polyuréthanes comprenant des groupes oxyalcoylène.

6. Compositions selon l'une des revendications 1 à 5, caractérisées en ce qu'elles vérifient:

$$m + p \geq 0{,}5$$

et

$$p + q \geq \frac{n}{100} - 2{,}5,$$

les lettres n, m, p, q désignant respectivement:

n: le nombre de milliéquivalents de motifs oxyéthylène $—O—CH_2—CH_2—$ pour 100 g de polyuréthane,

m: le nombre de milliéquivalents de groupes amine tertiaire, salifiés ou non, pour 100 g de polyuréthane,

p: le nombre de milliéquivalents de groupes ammonium quaternaire pour 100 g de polyuréthane,

q: le nombre de milliéquivalents de groupes ioniques pour 100 g de polymère du chlorure de vinyle.

7. Compositions selon l'une des revendications 1 à 6, caractérisées en ce que les polyétheruréthanes comprennent une pluralité de motifs récurrents de formule:

$$—A—NH—CO—O—B—O—CO—NH— \quad (I)$$

et des motifs récurrents et formule:

$$—A—NH—CO—Z—NH— \quad (II)$$

dans lesquelles:

— B est le reste divalent d'un macrodiol de type polyéther de formule: HO—B—OH

— A est le reste divalent d'un diisocyanate aliphatique et/ou cycloaliphatique et/ou aromatique de formule; O=C=N—A—N=C=O,

— Z est un lien valentiel ou un radical divalent tel que:

$$—NH—NH—CO—;$$

$$—NH—CH_2—CO—NH—NH—CO—;$$

ou

$$—NR_2—D—NR_3—CO—$$

ou

$$—O—M—O—CO—;$$

$R_2$, $R_3$ et M sont tels que $NHR_2—D—NHR_3$ soit une diamine primaire ou secondaire et HO—M—OH un diol, dans lesquels:

— $R_2$ et $R_3$, identiques ou différents, sont des atomes d'hydrogène ou des radicaux alcoyles inférieurs,

— D est une chaîne hydrocarbonée renfermant de 2 à 12 atomes de carbone, un cycle hydrocarboné à 5 ou 6 atomes de carbone ou un hétérocycle azoté à 5 ou 6 maillons renfermant 1 ou 2 atomes d'azote, les chaînes et cycles hydrocarbonés étant saturés ou insaturés, non substitués ou substitués par un ou deux radicaux alcoyles inférieurs ou par un radical dialcoylamino ou par un hétérocycle azoté à 5 ou 6 chaînons rattaché par un atome d'azote, deux des chaînes ou cycles pouvant être non substitués ou substitués, en particulier sur un atome d'azote, par un radical alcoyle inférieur,

— M est un radical hydrocarboné aliphatique renfermant 2 à 12 atomes de carbone, linéaire ou ramifié, saturé ou à insaturation éthylénique ou acétylénique, non substitué ou substitué par un ou deux radicaux alcoyles inférieurs ou par un radical dialcoylamino, ininterrompu ou interrompu par un radical alcoylimino.

8. Objets conformés caractérisés en ce qu'ils sont constitués en tout ou partie de compositions selon l'une des revendications 1 à 7.

9. Procédé de préparation d'objets conformés selon la revendication 8, et notamment de tuyaux et cathéters, caractérisé en ce qu'un support est enduit à l'aide d'une solution d'une composition selon l'une des revendications 1 à 7 et que cette solution enduite est évaporée.

10. Procédé de préparation d'objets conformés selon la revendication 8, notamment sous forme de tuyaux ou catheters, caractérisé en ce que l'on extrude à une température inférieure à 100°C une pâte à base de compositions selon l'une des revendications 1 à 8.

11. Compositions polymériques selon l'une quelconque des revendications 1 à 7, caractérisées en ce que le polyuréthane comprend des groupes ammonium, de préférence quaternaire, et en ce qu'elles comprennent de l'héparine.

12. Compositions selon la revendication 11, caractérisées en ce que $p_1 - q_1 > 0$ et que la proportion d'héparine par rapport à l'ensemble des polymères est comprise entre 0,5 et 35% en poids, $p_1$ étant le produit de p que multiplie le pourcentage pondéral du polyuréthane dans le mélange divisé par 100, $q_1$ étant le produit de q que multiplie le pourcentage pondéral du polymère de chlorure de vinyle à sites ioniques dans le mélange divisé par 100.

13. Compositions selon l'une des revendications 11 et 12, caractérisées en ce que la proportion d'héparine par rapport aux polymères est comprise entre 1 et 15%.

14. Objets conformés, caractérisés en ce qu'ils sont constitués en tout ou partie de compositions selon l'une des revendications 11 à 13.

15. Procédé de préparation de produits selon l'une des revendications 11 à 14, caractérisé en ce que l'on mélange deux solutions, la première étant une solution du polymère du chlorure de vinyle et la

seconde étant une solution d'héparine et du polyuréthane ou bien, la première étant une solution d'héparine et la seconde étant une solution de polymère du chlorure de vinyle et du polyuréthane, puis que l'on évapore cette solution, éventuellement après enduction d'un support par cette dernière.

16. Procédé de préparation de produits selon l'une des revendications 11 à 14, caractérisé en ce qu'on extrude à température inférieure à 100°C une pâte comprenant un agent gonflant ou un solvant et une composition selon l'une des revendications 11 à 13, cette pâte ayant été obtenue à partir d'une solution contenant l'héparine et le polyétheruréthane et éventuellement le polymère du chlorure de vinyle, cette solution ayant été évaporée ou précipitée avec un non solvant, éventuellement un solvant volatil ayant été ajouté au précipité, et aussi éventuellement, le polymère du chlorure de vinyl étant malaxé avec la pâte si cette dernière ne le contient pas déjà.

17. Procédé selon l'une des revendications 15 ou 16, caractérisé en ce que les solutions ou pâte contenant de l'héparine contiennent également du diméthylsulfoxyde ou du diéthylèneglycol ou du diméthylformamide ou de l'eau.

18. Objets conformés selon la revendication 8, dans lesquels le polyétheruréthane utilisé comprend des groupes ammonium, de préférence quaternaire, caractérisés en ce qu'ils sont héparinés après leur mise en forme.

**Patentansprüche**

1. Homogene, polymere Zusammensetzungen, welche insbesondere zur Herstellung von biegsamen, verformten Erzeugnissen verwendbar sind, die im allgemeinen transparent sind und eine gute Verträglichkeit mit biologischen und physiologischen Flüssigkeiten haben, dadurch gekennzeichnet, daß sie derart sind, daß sie erhalten werden, indem ein Polyetherurethan mit einer tertiären Amino- und/oder Ammoniumgruppe wobei das Polyetherurethan keine Polyester-Verkettungen trägt, und einem Vinylchloridpolymeren vermischt wurde,

— wobei dieses Gemisch erhalten wurde, indem die beiden Polymeren in einem Lösungsmittelmedium vollständig gelöst wurden und dann das Lösungsmittel verdampft wurde,

— oder das Gemisch wird erhalten, ausgehend von einer Paste mit einem Quellmittel oder einer Lösungsmittelmenge, die nicht ausreicht, um die Gesamtheit der Polymeren zu lösen, und daß man dann diese Paste extrudiert.

2. Zusammensetzungen gemäß Anspruch 1, dadurch gekennzeichnet, daß das Mengenverhältnis von Polyurethan zwischen 20 und 60 Gew.% liegt.

3. Zusammensetzungen gemäß einem der Ansprüche 1 bis 2, dadurch gekennzeichnet, daß das Vinylchloridpolymere wenigstens 60 Gew.% Chlorethylen-Gruppierungen umfaßt.

4. Zusammensetzungen gemäß einem der Ansprüche 1 bis 3, dadurch gekennzeichnet, daß das Vinylchloridpolymere ein Homopolymeres des Vinylchlorids oder ein Vinylchlorid/Vinylester-Copolymeres ist.

5. Zusammensetzungen gemäß einem der Ansprüche 1 bis 4, dadurch gekennzeichnet, daß die Polyetherurethane Polyurethane mit Oxyalkylengruppen sind.

6. Zusammensetzungen gemäß einem der Ansprüche 1 bis 5, dadurch gekennzeichnet, daß gilt:

$$m + p \geq 0,5$$

und

$$p + q \geq \frac{n}{100} - 2,5,$$

wobei die Buchstaben n, m, p und q folgende Bedeutungen besitzen:

n: die Zahl der Milliäquivalente an Oxyethylen-Gruppierungen $—O—CH_2—CH_2—$ für 100 g Polyurethan;

m: die Zahl der Milliäquivalente an tertiären Amingruppen, als Salz oder nicht, für 100 g Polyurethan;

p: die Zahl der Milliäquivalente an Ammoniumgruppen für 100 g Polyurethan;

q: die Zahl der Milliäquivalente an ionischen Gruppen für 100 g Polymeres des Vinylchlorids.

7. Zusammensetzungen gemäß einem der Ansprüche 1 bis 6, dadurch gekennzeichnet, daß die Polyurethane umfassen: eine Vielzahl von widerkehrenden Gruppierungen der Formel I

$$—A—NH—CO—O—B—O—CO—NH— \quad (I)$$

und wiederkehrenden Gruppierungen der Formel II

$$—A—NH—CO—Z—NH— \quad (II)$$

worin bedeuten:

B den zweiwertigen Rest eines Makrodiols vom Polyether-Typ der Formel: HO—B—OH;

A den zweiwertigen Rest eines aliphatischen und/oder cycloaliphatischen und/oder aromatischen Di-isocyanats der Formel: O=C=N—A—N=C=O;

Z eine Valenzbindung oder einen zweiwertigen Rest, wie

—NH—NH—CO—;

$$-NH-CH_2-CO-NH-NH-CO-;$$

oder

$$-NR_2-D-NR_3-CO-$$

oder

—O—M—O—CO—;

$R_2$, $R_3$ und M sind beispielsweise wie entweder ein primäres oder sekundäres Diamin $NHR_2-D-NHR_3$ und ein Diol HO—M—OH, worin bedeuten:

$R_2$ und $R_3$, die identisch oder verschieden sind, Wasserstoffatome oder niedermolekulare Alkylreste;

D eine Kohlenwasserstoffkette mit 2 bis 12 Kohlenstoffatomen, einen Kohlenwasserstoffring mit 5 oder 6 Kohlenstoffatomen oder einen Stickstoffheterocyclus mit 5 oder 6 Gliedern mit 1 oder 2 Stickstoffatomen, wobei die Kohlenwasserstoffketten und -ringe gesättigt oder ungesättigt, nicht substituiert oder substituiert sind durch einen oder zwei niedermolekulare Alkylreste oder durch einen Dialkylaminorest oder durch einen Stickstoffheterocyclus mit 5 oder 6 Gliedern, verknüpft durch ein Stickstoffatom, wobei zwei der Ketten oder Ringe untereinander durch eine Alkyliminogruppe verbunden sein können; wobei die Heterocyclen nicht substituiert oder substituiert, besonders am Stickstoffatom durch einen niedermolekularen Alkylrest, sein können,

M einen geradkettigen oder verzweigten, aliphatischen Kohlenwasserstoffrest mit 2 bis 12 Kohlenstoffatomen, der gesättigt ist oder mit ethylenischer oder acetylenischer Ungesättigtheit, der nicht substituiert ist oder substituiert ist durch einen oder zwei niedermolekulare Alkylreste oder durch einen Dialkylaminorest, der ununterbrochen oder durch einen Alkyliminorest unterbrochen ist.

8. Geformte Erzeugnisse, dadurch gekennzeichnet, daß sie ganz oder teilweise aus Zusammensetzungen gemäß einem der Ansprüche 1 bis 7 bestehen.

9. Verfahren zur Herstellung von geformten Erzeugnissen gemäß Anspruch 8 und insbesondere von Röhren und Kathetern, dadurch gekennzeichnet, daß ein Träger mit Hilfe einer Lösung einer Zusammensetzung gemäß einem der Ansprüche 1 bis 7 überzogen wird und daß diese überzogene Lösung abgedampft wird.

10. Verfahren zur Herstellung von geformten Gegenständen gemäß Anspruch 8, insbesondere in Form von Röhren oder Kathetern, dadurch gekennzeichnet, daß man bei einer Temperatur unterhalb 100°C eine Paste auf Basis der Zusammensetzungen gemäß einem der Ansprüche 1 bis 8 extrudiert.

11. Polymere Zusammensetzungen gemäß einem der Ansprüche 1 bis 7, dadurch gekennzeichnet, daß das Polyurethan Ammoniumgruppen, vorzugsweise quaternäre, umfaßt und daß sie Heparin beinhalten.

12. Zusammensetzungen gemäß Anspruch 11, dadurch gekennzeichnet, daß $p_1 - q_1 > 0$ ist und daß das Heparin-Mengenverhältnis in bezug auf die Gesamtheit der Polymeren zwischen 0,5 und 35 Gew.% liegt, wobei $p_1$ das Produkt von p ist, das die Gewichtsprozent des Polyurethans in dem Gemisch, geteilt durch 100, multipliziert, $q_1$ das Produkt von q ist, das die Gewichtsprozent des Vinylchloridpolymeren mit ionischen Stellen in dem Gemisch, geteilt durch 100, multipliziert.

13. Zusammensetzungen gemäß einem der Ansprüche 11 und 12, dadurch gekennzeichnet, daß das Heparin-Mengenverhältnis in Bezug auf die Polymeren zwischen 1 und 15% liegt.

14. Geformte Erzeugnisse, dadurch gekennzeichnet, daß sie ganz oder teilweise aus Zusammensetzungen gemäß einem der Ansprüche 11 bis 13 bestehen.

15. Verfahren zur Herstellung von Produkten gemäß einem der Ansprüche 11 bis 14, dadurch gekennzeichnet, daß man zwei Lösungen vermischt, wobei die erste eine Lösung des Polymeren des Vinylchlorids ist und die zweite eine Heparinlösung und des Polyurethans ist oder die erste eine Heparinlösung und die zweite eine Lösung des Polymeren des Vinylchlorids und des Polyurethans ist, und daß man diese Lösung dann verdampft, gegebenenfalls nach Überziehen eines Trägers mit dieser letzteren.

16. Verfahren zur Herstellung von Produkten gemäß einem der Ansprüche 11 bis 14, dadurch gekennzeichnet, daß man bei einer Temperatur unterhalb 100°C eine Paste extrudiert, welche ein Quellmittel oder ein Lösungsmittel und eine Zusammensetzung gemäß einem der Ansprüche 11 bis 13 enthält, wobei diese Paste erhalten wurde, ausgehend von einer Lösung, die das Heparin und das Polyetherurethan und gegebenenfalls das Polymere des Vinylchlorids enthält, wobei diese Lösung verdampft oder mit einem Nicht-Lösungsmittel ausgefällt wurde, wobei gegebenenfalls ein flüchtiges Lösungsmittel zu dem Niederschlag gegeben wurde und wobei gegebenenfalls das Polymere des Vinylchlorids mit der Paste verknetet wird, wenn diese letztere es nicht bereits enthält.

17. Verfahren gemäß einem der Ansprüche 15 oder 16, dadurch gekennzeichnet, daß die Lösungen oder die Paste, welche Heparin enthalten, auch Dimethylsulfoxid oder Diethylenglykol oder Dimethylformamid oder Wasser enthalten.

18. Geformte Gegenstände gemäß Anspruch 8, in denen das verwendete Polyurethan Ammoniumgruppen, vorzugsweise quaternäre, umfaßt, dadurch gekennzeichnet, daß sie nach ihrer Formgebung heparinisiert werden.

**Claims**

1. Homogeneous polymer compositions which can be used, in particular, for producing flexible shaped articles which are generally transparent and have a good compatibility with biological and physiological liquids, the said compositions being characterised in that they are like those obtained by mixing a polyether-urethane containing a tertiary amine and/or ammonium group, the said polyether-urethane not containing a polyester linkage, with a vinyl chloride polymer,

— when this mixture is obtained by completely dissolving the two polymers in a solvent medium and then evaporating off the solvent, or

— when the mixture is obtained from a paste with a blowing agent or an insufficient amount of solvent to dissolve the total amount of the polymers, this paste then being extruded.

2. Compositions according to Claim 1, characterised in that the proportion of polyurethane is between 20 and 60% by weight.

3. Compositions according to one of Claims 1 to 2, characterised in that the vinyl chloride polymer comprises at least 60% by weight of chloroethylene units.

4. Compositions according to one of Claims 1 to 3, characterised in that the vinyl chloride polymer is a vinyl chloride homopolymer or a vinyl chloride/vinyl ester copolymer.

5. Compositions according to one of Claims 1 to 4, characterised in that the polyether-urethanes are polyurethanes comprising oxyalkylene groups.

6. Compositions according to one of Claims 1 to 5, characterised in that they satisfy:

$$m + p \geq 0.5$$

and

$$p + q \geq \frac{n}{100} - 2.5,$$

the letters n, m, p and q respectively denoting:

n: the number of milliequivalents of oxyethylene units $—O—CH_2—CH_2—$ per 100 g of polyurethane,

m: the number of milliequivalents of tertiary amine groups, which may or may not be salified, per 100 g of polyurethane,

p: the number of milliequivalents of quaternary ammonium groups per 100 g of polyurethane, and

q: the number of milliequivalents of ionic groups per 100 g of vinyl chloride polymer.

7. Compositions according to one of Claims 1 to 6, characterised in that the polyether-urethanes comprise a plurality of repeat units of the formula:

$$—A—NH—CO—O—B—O—CO—NH— \quad (I)$$

and of repeat units of the formula:

$$—A—NH—CO—Z—NH— \quad (II)$$

in which formulae:

— B is the divalent radical of a macrodiol of the polyether type, of the formula: HO—B—OH

— A is the divalent radical of an aliphatic and/or cycloaliphatic and/or aromatic diisocyanate of the formula: O=C=N—A—N=C=O,

— Z is a valence bond or a divalent radical such as:

$$—NH—NH—CO—,$$

$$—NH—CH_2—CO—NH—NH—CO—$$

or

$$—NR_2—D—NR_3—CO—$$

or

$$—O—M—O—CO—;$$

$R_2$, $R_3$ and M are such that $NHR_2—D—NHR_3$ is a primary or secondary diamine and HO—M—OH is a diol, in which formulae:

— $R_2$ and $R_3$, which are identical or different, are hydrogen atoms or lower alkyl radicals,

— D is a hydrocarbon chain containing from 2 to 12 carbon atoms, a hydrocarbon ring containing 5 or 6 carbon atoms or a 5-membered or 6-membered nitrogen-containing heterocyclic ring containing 1 or 2 nitrogen atoms, the hydrocarbon chains and rings being saturated or unsaturated and unsubstituted or substituted by one or two lower alkyl radicals, by a dialkylamino radical or by a 5-membered or 6-membered nitrogen-containing heterocyclic ring attached by a nitrogen atom, it being possible for two of the chains or rings to be joined to one another by an alkylimino group and for the heterocyclic rings to be unsubstituted or substituted, in particular on a nitrogen atom, by a lower alkyl radical, and

— M is an aliphatic hydrocarbon radical containing 2 to 12 carbon atoms, which is linear or branched, saturated or ethylenically or acetylenically unsaturated, unsubstituted or substituted by one or two lower alkyl radicals or by a dialkylamino radical, and uninterrupted or interrupted by an alkylimino radical.

8. Shaped articles, characterised in that they totally or partially consist of compositions according to one of Claims 1 to 7.

9. Process for the preparation of shaped articles according to Claim 8, and in particular of tubes and catheters, characterised in that a support is coated with a solution of a composition according to one of Claims 1 to 7, and in that this coated solution is evaporated.

10. Process for the preparation of shaped articles according to Claim 8, in particular in the form of tubes or catheters, characterised in that a paste based on compositions according to one of Claims 1 to 8 is extruded at a temperature below 100°C.

11. Polymer compositions according to any one of Claims 1 to 7, characterised in that the polyurethane comprises ammonium groups, preferably quaternary ammonium groups, and in that they comprise heparin.

12. Compositions according to Claim 11, characterised in that $p_1 - q_1 > 0$ and in that the proportion of heparin is between 0.5 and 35% by weight, relative to all the polymers, $p_1$ being the product of p multiplied by the percentage by weight of the polyurethane in the mixture, divided by 100, and $q_1$ being the product of q multiplied by the percentage by weight of the vinyl chloride polymer with ionic sites, in the mixture, divided by 100.

13. Compositions according to one of Claims 11 and 12, characterised in that the proportion of heparin is between 1 and 15%, relative to the polymers.

14. Shaped articles, characterised in that they totally or partially consist of compositions according to one of Claims 11 to 13.

15. Process for the preparation of products according to one of Claims 11 to 14, characterised in that two solutions are mixed, the first being a solution of the vinyl chloride polymer and the second being a solution of heparin and of the polyurethane, or alternatively the first being a solution of heparin and the second being a solution of vinyl chloride polymer and of the polyurethane, and in that this solution is then evaporated, optionally after a support has been coated with the latter.

16. Process for the preparation of products according to one of Claims 11 to 14, characterised in that a paste, comprising a blowing agent or a solvent and a composition according to one of Claims 11 to 13, is extruded at a temperature below 100°C, this paste having been obtained from a solution containing heparin and the polyetherurethane and optionally the vinyl chloride polymer, this solution having been evaporated or precipitated with a non-solvent, a volatile solvent having optionally been added to the precipitate and, also optionally, the vinyl chloride polymer being malaxated with the paste if the latter does not already contain the said polymer.

17. Process according to one of Claims 15 or 16, characterised in that the solutions or paste containing the heparin also contain dimethyl sulphoxide, diethylene glycol, dimethyl formamide or water.

18. Shaped articles according to Claim 8, in which the polyether-urethane used comprises ammonium groups, preferably quaternary ammonium groups, characterised in that they are treated with heparin after they have been shaped.